# NEUE EUROPÄISCHE PATENTSCHRIFT

(11) **EP 1 982 741 B2**
(45) Veröffentlichungstag und Bekanntmachung des Hinweises auf die Entscheidung über den Einspruch: **14.09.2016**
(45) Hinweis auf die Patenterteilung: 23.06.2010
(21) Anmeldenummer: 07022312.8
(22) Anmeldetag: 16.11.2007
(51) Int. Cl.: A61M 25/00, A61M 25/01

(54) **Katheter-Set**
Catheter set
Ensemble de cathéter

(30) Priorität: 18.04.2007 DE 102007018277
(43) Veröffentlichungstag der Anmeldung: 22.10.2008
(73) Patentinhaber: Medical Service GmbH, 75378 Bad Liebenzell (DE)
(72) Erfinder:
(74) Vertreter: Grünecker Patent- und Rechtsanwälte PartG mbB

(56) Entgegenhaltungen:
- EP-A1- 0 378 183
- WO-A-98/11932
- WO-A1-99/30761
- WO-A1-2006/009509
- DE-A1- 2 317 839
- DE-A1- 10 213 411
- DE-A1- 10 334 372
- GB-A- 2 033 231
- US-A- 3 345 988
- US-A- 3 648 704
- US-A1- 2003 139 730
- US-A1- 2005 043 715
- US-A1- 2005 109 648
- US-A1- 2005 199 521

## Beschreibung

Die Erfindung betrifft ein Katheter-Set, umfassend einen Blasenkatheter mit einem flexiblen Katheterschaft und einer Katheterspitze, und eine längliche Verpackung, in die der Blasenkatheter in einen abgeschlossenen Katheteraufnahmeraum eingelegt ist, wobei an das katheterspitzenseitige Ende des Katheteraufnahmeraums angrenzend von der Verpackung ein Reservoir mit einem Aktivierungsmittel und/oder einem Gleitmittel ausgebildet ist.

Ein derartiges Katheter-Set, wie es beispielsweise aus der US 4,269,310 bekannt geworden ist, erlaubt eine sterile Verpackung eines Blasenkatheters, der zum Gebrauch durch eine Austrittsstelle am katheterspitzenseitigen Ende eines von der Verpackung ausgebildeten Katheteraufnahmeraums aus der Verpackung geführt wird. Der Blasenkatheter tritt mit der Katheterspitze voran aus der Verpackung und wird direkt in die Harnröhre zur Harnblase eines Patienten geführt. Durch die gebrauchsfertige Lage des Blasenkatheters innerhalb des Katheteraufnahmeraums der Verpackung und durch die Flexibilität des Verpackungsmaterials wird ein unmittelbarer Kontakt zwischen dem Blasenkatheter und den ihn haltenden bzw. führenden Händen weitgehend vermieden. In der Verpackung ist ein von der Verpackung ausgebildetes Reservoir an das katheterspitzenseitige Ende des Katheteraufnahmeraums angrenzend angeordnet. In dem Reservoir ist ein Oberflächenaktivierungsmittel und/oder ein Gleitmittel vorgehalten. Dieses Reservoir wird vor dem Einführen des Katheters in die Harnröhre zur Harnblase eines Patienten aufgedrückt, so dass das Oberflächenaktivierungsmittel auf der Oberfläche des Blasenkatheters zu dessen Benetzung verteilt werden kann, um dessen Gleiteigenschaften zu verbessern. Die in der US 4,269,310 offenbarte Verpackung weist einen Aufreißstreifen zu deren Öffnung auf. Sie ist also aus verschiedenen Materialien hergestellt und daher relativ aufwändig herzustellen.

Die gattungsbildende US 3648704 A offenbart eine Katheterverpackung, wobei der Katheter In einem Katheteraufnahmeraum platziert ist und sich am katheterspitzenseitigen Ende der Verpackung ein Gleitmittel- bzw. Benetzungsmittelreservoir anschließt. Das Reservoir ist vom Katheteraufnahmeraum durch eine Schweißnaht getrennt. Zur Anwendung des Katheters wird das Reservoir über eine Perforationslinie von der Katheterverpackung getrennt. Die Katheterverpackung wird geöffnet, um den Katheter freizulegen, woraufhin das Benetzungsmittel auf den Katheter aufgebracht werden kann.

Der Erfindung liegt die Aufgabe zugrunde, ein Katheter-Set bereitzustellen, welches die Nachteile des Standes der Technik vermeidet, wobei insbesondere eine besonders einfache und kostengünstige Herstellung des Katheter-Sets, bei zumindest gleichbleibendem Anwendungskomfort, möglich sein soll.

Diese Aufgabe wird durch das Katheter-Set des unabhängigen Anspruchs gelöst. Die abhängigen Ansprüche stellen bevorzugte Ausführungsformen der Erfindung dar.

Ein erfindungsgemäßes Katheter-Set gemäß Anspruch 1 umfasst einen Blasenkatheter mit einem flexiblen Katheterschaft und einer Katheterspitze, und eine längliche Verpackung, in die der Blasenkatheter in einen abgeschlossenen, sterilen Katheteraufnahmeraum eingelegt Ist. An das katheterspitzenseitige Ende des Katheteraufnahmeraums angrenzend ist von der Verpakkung ein Reservoir mit einem Aktivierungsmittel und/ oder einem Gleitmittel ausgebildet. Erfindungsgemäß Ist die Verpackung aus einem Kunststofffolienschlauch hergestellt. Dabei sind das Reservoir und der Katheteraufnahmeraum durch bevorzugt senkrecht oder im Wesentlichen senkrecht zur Längsrichtung der Kunststofffolienverpackung verlaufende Kunststoffschweißnähte ausgebildet.

Die gesamte Verpackung ist also aus einem Kunststofffollenschlauch hergestellt, wobei die einzelnen zur Aufnahme des Blasenkatheters oder zur Aufnahme des Aktivierungsmittels und/oder des Gleitmittels vorgesehenen Abteilungen In der Verpackung durch einfaches Verschweißen von Wandbereichen der Kunststofffoltenverpackung hergestellt sind. Die Kunststofffolienverpeckung kann z.B. aus Polyethylen hergestellt sein. Die Kunststoffschweißnähte sind auf einfachste Welse mittels Erhitzen, entsprechend der Verschweißung von Lebensmittelverpackungen, herstellbar. Dadurch dass die gesamte Struktur der Verpackung lediglich durch derartige Kunststoffschweißnähte an einer Kunststofffolienverpackung hergestellt sind, wird eine besonders einfache und kostengünstige Herstellung des Katheter-Sets ermöglicht. Weil ein Kunststofffolienschlauch verwendet wird, ist kein Verschließen des Katheteraufnahmeraum und des Reservoirs in Längsrichtung der Verpackung bzw. des Blasenkatheters notwendig. Der Kunststofffolienschlauch besteht bereits aus einer in dessen Querrichtung umlaufend zusammenhängenden Kunststofffolie. Der Blasenkatheter wird einfach in die Kunststofffolienverpackung eingeschoben, woraufhin der Katheteraufnahmeraum unter Herstellung von zwei diesen abschließenden Kunststoffschweißnähten verschlossen wird. Daraufhin wird das Aktivierungsmittel und/oder das Gleitmittels in die Kunststofffolienverpackung eingefüllt und das Reservoir mittels einer weiteren Kunststoffschweißnaht verschlossen. Es kann auch umgekehrt vorgegangen werden und zunächst das Aktivierungsmittel und/oder das Gleitmittel eingefüllt werden und daraufhin der Blasenkatheter eingeschoben werden.

Wenn das Reservoir durch eine erste Kunststoffschweißnaht, die das Reservoir vom Katheteraufnahmeraum abgrenzt und eine zweite Kunststoffschweißnaht, die das Reservoir an einem freien Ende der Verpackung abschließt, ausgebildet ist und die zweite Kunststoffschweißnaht gegenüber der ersten Kunststoffschweißnaht verstärkt ausgebildet ist, platzt bei Drücken auf das Reservoir zu dessen Öffnung lediglich die erste Kunststoffschweißnaht auf, wodurch das darin enthaltene Aktivierungsmittel und/oder Gleitmittel zuverlässig in den Katheteraufnahmeraum gepresst werden kann.

Bevorzugt ist an der Verpackung ein Haltebereich seitlich im Bereich des katheterspitzenseitigen Endes des Katheteraufnahmeraums ausgebildet, durch den der Katheteraufnahmeraum in Richtung des Reservoirs zur Ausbildung eines Austrittskanals für den Blasenkatheter verjüngt ist. Auch dieser Haltebereich ist durch eine Kunststoffschweißnaht an der die Verpakkung ausbildenden Kunststofffolienverpackung ausgebildet. Dadurch wird eine Positionierung der Katheterspitze bei dessen Applikation erleichtert. Weiter kann derart eine Führung des Aktivierungsmittels und/oder Gleitmittels nach dem Öffnen des Reservoirs zur Katheterspitze hin unterstützt werden. Die Benetzung der Katheteroberfläche mit Aktivierungsmittel und/oder Gleitmittel wird¹so erleichtert.

Vorteilhaft ist der Haltebereich derart von einer dritten Kunststoffschweißnaht gebildet, dass die dritte Kunststoffschweißnaht ein gegenüberdem Katheteraufnahmeraum luftdichtes Haltebereichlumen ausbildet, wobei im Material der das Haltebereichlumen bildenden Kunststoffföllenverpackung eine bevorzugt als eine Perforierung ausgebildete Sollbruchstelle zum Abtrennen des das Reservoir ausbildenden Materials der Kunststofffolienverpackung ausgebildet ist Durch das Ermöglichen des einfachen Abtrennens des Reservoirs kann die das Reservoir nach außen abschließende Kunststoffschweißnaht besonders.stark ausgeführt sein, ohne dass ein Herausschieben des Blasenkatheters aus der Verpackung erschwert wird. Weiter wird die Applikation des Blasenkatheters nicht durch ein während der Applikation an der Verpackung verbleibendes leeres Reservoir erschwert.

Bevorzugt ist im Material der das Haltebereichlumen bildenden Kunststofffolienverpackung eine weitere bevorzugt als eine Perforierung ausgebildete Sollbruchstelle zum Abtrennen des sich an den Haltebereich in Richtung des katheterspitzenfernen Endes des Blasenkatheters anschließenden den Katheteraufnahmeraum ausbildenden Materials der Kunststofffolienverpackung ausgebildet. Dadurch kann die Verpackung während der Applikation des Blasenkatheters weitestgehend entferntwerden. Letzteres kann z.B. nach hälftigem Einführen des Blasenkatheter in die Harnröhre vom den Blasenkatheter applizierenden Patienten gewünscht sein. Dabei verbleibt der Teil der Verpackung, der den Haltebereich ausbildet, weiterhin am Blasenkatheter um eine Kontamination des Blasenkatheters durch Berühren mit der Hand zu verhindern.

Wenn seitlich im Bereich des katheterspitzenfernen Endes des Katheteraufnahmeraums an der Verpackung ein gegenüber dem Katheteraufnahmeraum luftdicht abgeschlossener Abtrennbereich von einer vierten Kunststoffschweißnaht ausgebildet ist und im Material der den Abtrennbereich bildenden Kunststofffollenverpackung eine bevorzugt als eine Perforierung ausgebildete Sollbruchstelle zum Abtrennen des das katheterspitzenferne Endes des Katheteraufnahmeraums ausbildenden Materials der Kunststofffolienverpackung ausgebildet ist, kann durch Abtrennen des das katheterspitzenferne Endes des Katheteraufnahmeraums ausbildenden Materials die Verpackung an deren unteren Ende problemlos geöffnet werden. Dadurch Ist es möglich, die Verpackung, d.h. den Katheteraufnahmeraum, als Verlängerung des Blasenkatheters zum Ableiten des Harns zu verwenden.

Vorteilhaft weist die Kunststofffolienverpakkung zumindest in Teilbereichen ihrer Oberfläche eine antimikrobielle Beschichtung, bevorzugt eine Silberbeschichtung, auf.
Wenn der Blasenkatheter an seinem katheterspitzenfernen Ende ein das Ende verdickendes Blockierelement aufweist, wird ein Herausrutschen des vollständigen Katheters aus dem Katheteraufnahmeraum, z.B. durch einen ausgebildeten Austrittskanal, verhindert. Das Blokkierelement kann auch als Trichter ausgeformt sein, wodurch ein Ableiten des Harns vereinfacht wird.

Die Erfindung wird nachfolgend anhand eines Ausführungsbeispiels unter Bezugnahme auf die Zeichnung näher erläutert.

Die Darstellungen der Zeichnung zeigen den erfindungsgemäßen Gegenstand stark schematisiert und sind nicht maßstäblich zu verstehen. Die einzelnen Bestandteile des erfindungsgemäßen Gegenstandes sind so dargestellt, dass ihr Aufbau gut gezeigt werden kann.

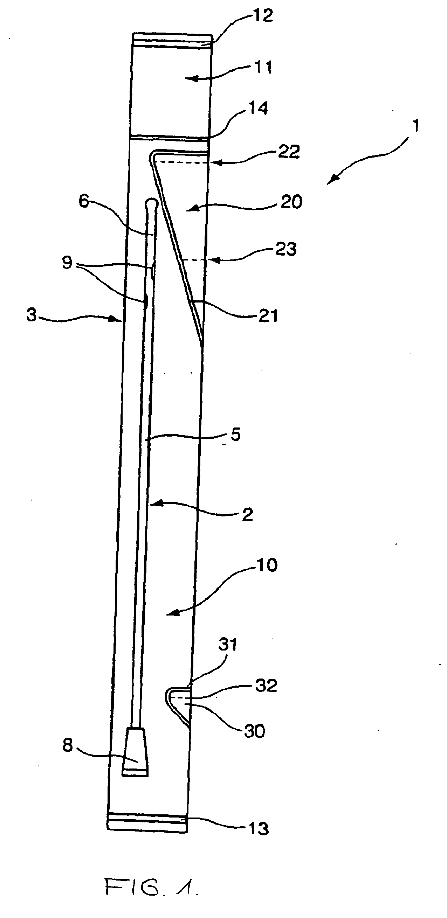

In Figur 1 ist ein erfindungsgemäßes Katheter-Set 1 dargestellt. Das Katheter-Set 1 weist einen Blasenkatheter 2 und eine Verpackung 3 aus einem Kunststofffolienschlauch auf. Der Blasenkatheter 2 besteht aus einem flexiblen Katheterschaft 5 und einer Katheterspitze 6 sowie einem, am katheterspitzenfernen Ende des Katheterschafts 5 angeordneten, dieses Ende verdickenden Blockierelement 8. Am Katheterschaft 5 sind zwei zur Ableitung von Harn dienende sogenannte Augen 9 vorhanden. Innerhalb der länglichen Verpakkung ist der Blasenkatheter 2 In einen Katheteraufnahmeraum 10 eingelegt. An das katheterspitzenseitige Ende des Katheteraufnahmeraums 10 angrenzend ist von der Verpackung ein Reservoir 11 mit einem Aktivierungsmittel und/oder einem Gleitmittel ausgebildet. Die Enden der Kunststofffolienverpackung sind zum luftdichten, bzw. aktivierungsmittel- und/oder einem gleitmitteldichten Verschluss der Verpackung 3 mittels Kunststoffschweißnähten 12,13 verschlossen.

Das Reservoir 11 ist durch zwei Kunststoffschweißnähte 12,14 im Kunststofffolienschlauch, aus dem die Verpackung 3 hergestellt ist, abgetrennt Eine erste Kunststoffschweißnaht 14 grenzt das Reservoir 11 vom Katheteraufnahmeraum 10 ab. Die zweite Kunststoffschweißnaht 12 schließt das Reservoir 11 an einem freien Ende der Verpackung 3 ab. Die zweite Kunststoffschweißnaht 12 Ist gegenüber der ersten Kunststoffschweißnaht 14 verstärkt ausgebildet Bei Drücken auf das Reservoir 11 platzt daher lediglich die erste Kunststoffschweißnaht 14 auf, wodurch das darin enthaltene Aktivierungsmittel und/oder Gleitmittel zuverlässig in den Katheteraufnahmeraum 10 gepresst werden kann.

An der Verpackung 3 ist seitlich im Bereich des katheterspitzenseitigen Endes des Katheteraufnahmeraums 10 ein Haltebereich 20 ausgebildet. Durch diesen Haltebereich 20 ist der Katheteraufnahmeraum 10 in Richtung des Reservoirs 11 verjüngt um einen Austrittskanal für den Blasenkatheter 2 auszuformen. Auch der Haltebereich 20 Ist durch eine Kunststoffschweißnaht 21 an dem die Verpackung 3 ausbildenden Kunststofffolienschlauch ausgebildet.
Durch diese Kunststoffschweißnaht 21 ist ein gegenüber dem Katheteraufnahmeraum 10 luftdichtes Haltebereichlumen ausgebildet. Im Material der das Halteberelchlumen bildenden Kunststofffolienverpackung ist eine Perforierung 22 ausgebildet, die als Sollbruchstelle zum Abtrennen des das Reservoir 11 ausbildenden Materials der Kunststofffolienverpackung dient. Um die Dichtigkeit des Katheteraufnahmeraums 10 zu gewährleisten, reicht die Perforierung 22 lediglich bis an den haltebereichlumenseitigen Rand der den Haltebereich 20 ausbildenden Kunststoffschweißnaht 21 heran. Im Material der das Haltebereichlumen bildenden Kunststofffolienverpackung ist eine weitere, als eine weitere Perforierung 23 ausgebildete Sollbruchstelle zum Abtrennen des sich an den Haltebereich 20 in Richtung des katheterspitzenfernen Endes des Blasenkatheters 2 anschließenden, den Katheteraufnahmeraum 10 ausbildenden Materials der Kunststofffolienverpackung ausgebildet Auch diese weitere Perforation 23 reicht lediglich bis an den haltebereichlumenseitigen Rand der den Haltebereich 20 ausbildenden Kunststoffschweißnaht 21.

Seitlich im Bereich des katheterspitzenfernen Endes des Katheteraufnahmeraums 10 ist an der Verpackung 3 ein gegenüber dem Katheteraufnahmeraum 10 luftdicht abgeschlossener Abtrennbereich 30 ausgebildet. Auch dieser Abtrennbereich 30 ist entsprechend dem Haltebereich 20 von einer Kunststoffschweißnaht 31 ausgebildet und weist eine als Perforierung 32 im Material der den Abtrennbereich 30 bildenden Kunststofffolienverpackung ausgebildete Sollbruchstelle auf, die zum Abtrennen des das katheterspitzenferne Ende des Katheteraufnahmeraums 10 ausbildenden Materials der Kunststofffolienverpackung dient

Vorgeschlagen wird ein Katheter-Set 1, umfassend einen Blasenkatheter 2 mit einem flexiblen Katheterschaff 5 und einer Katheterspitze 6, und eine längliche Verpackung 3, in die der Blasenkatheter 2 in einen abgeschlossenen Katheteraufnahmeraum 10 eingelegt ist, wobei an das katheterspitzenseitige Ende des Katheteraufnahmeraums 10 angrenzend von der Verpackung 3 ein Reservoir 11 mit einem Aktivierungsmittel und/oder einem Gleitmittel ausgebildet Ist. Die Verpackung 3 ist aus einem Kunststofffolienschlaucht hergestellt, wobei das Reservoir 11 und der Katheteraufnahmeraum 10 durch bevorzugt im Wesentlichen senkrecht zur Längsrichtung der Kunststofffollenverpackung verlaufende Kunststoffschweißnähte 12,13,14 ausgebildet sind.

Die Erfindung beschränkt sich nicht auf die vorstehend angegebenen Ausführungsbeispiele. Vielmehr ist eine Anzahl von Varianten denkbar, welche auch bei grundsätzlich anders gearteter Ausführung von den Merkmalen der Erfindung Gebrauch machen.

## Patentansprüche

1. Katheter-Set (1), umfassend einen Blasenkatheter (2) mit einem flexiblen Katheterschaft (5) und einer Katheterspitze (6), und eine längliche Kunststofffolienverpackung (3), in die der Blasenkatheter (2) in einen abgeschlossenen Katheteraufnahmeraum (10) eingelegt ist, wobei an das katheterspitzenseitige Ende des Katheteraufnahmeraums (10) angrenzend von der Verpackung (3) ein Reservoir (11) mit einem Aktivierungsmittel und/oder einem Gleitmittel ausgebildet ist, wobei das Reservoir (11) und der Katheteraufnahmeraum (10) durch bevorzugt im Wesentlichen senkrecht zur Längsrichtung der Kunststofffolienverpackung verlaufende Kunststoffschweißnähte (12,13,14) ausgebildet sind, und das Reservoir (11) durch eine erste Kunststoffschweißnaht (14), die das Reservoir (11) vom Katheteraufnahmeraum (10) abgrenzt und eine zweite Kunststoffschweißnaht (12), die das Reservoir (11) an einem freien Ende der Verpackung (3) abschließt, ausgebildet ist, **dadurch gekennzeichnet, dass** die Kunststofffolienverpackung (3) aus einem Kunststofffolienschlauch hergestellt ist, dass die zweite Kunststoffschweißnaht (12) gegenüber der ersten Kunststoffschweißnaht (14) verstärkt ausgebildet ist, so dass bei Drücken auf das Reservoir (11) lediglich die erste Kunststoffschweißnaht (14) zum Öffnen des Reservoirs (11) aufplatzt.

2. Katheter-Set nach Anspruch 1, **dadurch gekennzeichnet, dass** an der Verpackung (3) ein Haltebereich (20) seitlich im Bereich des katheterspitzenseitigen Endes des Katheteraufnahmeraums (10) ausgebildet ist durch den der Katheteraufnahmeraum (10) in Richtung des Reservoirs (11) zur Ausbildung eines Austrittskanals für den Blasenkatheter (2) verjüngt ist, wobei der Haltebereich (20) durch eine dritte Kunststoffschweißnaht (21) am die Verpackung (3) ausbildenden Kunststofffolienschlauch ausgebildet ist.

3. Katheter-Set nach Anspruch 2, **dadurch gekennzeichnet, dass** der Haltebereich (20) derart von der dritten Kunststoffschweißnaht (21) gebildet ist, dass die dritte Kunststoffschweißnaht (21) ein gegenüber dem Katheteraufnahmeraum (10) luftdichtes Haltebereichlumen ausbildet, wobei im Material der das Haltebereichlumen bildenden Kunststofffolienverpackung eine bevorzugt als eine Perforierung (22) ausgebildete Sollbruchstelle zum Abtrennen des das Reservoir (11) ausbildenden Materials der Kunststofffolienverpackung ausgebildet ist.

4. Katheter-Set nach Anspruch 3, **dadurch gekennzeichnet dass** im Material der das Haltebereichlumen bildenden Kunststofffolienverpackung eine weitere bevorzugt als eine Perforierung (23) ausgebildete Sollbruchstelle zum Abtrennen des sich an den Haltebereich (20) in Richtung des katheterspitzenfernen Endes des Blasenkatheters (2) anschließenden des Katheteraufnahmeraums (20) ausbildenden Materials der Kunststofffolienverpackung ausgebildet ist.

5. Katheter-Set nach einem der Ansprüche 1 bis 4, **dadurch gekennzeichnet, dass** seitlich im Bereich des katheterspitzenfernen Endes des Katheteraufnahmeraums (10) an der Verpackung (3) ein gegenüber dem Katheteraufnahmeraum (10) luftdicht abgeschlossener Abtrennbereich (30) von einer vierten Kunststoffschweißnaht (31) ausgebildet ist, wobei im Material der den Abtrennbereich (30) bildenden Kunststofffolienverpackung eine bevorzugt als eine Perforierung (32) ausgebildete Sollbruchstelle zum Abtrennen des das katheterspitzenferne Endes des Katheteraufnahmeraums (10) ausbildenden Materials der Kunststofffolienverpackung ausgebildet ist.

6. Katheter-Set nach einem der Ansprüche 1 bis 5, **dadurch gekennzeichnet, dass** der Kunststofffolienschlauch zumindest in Teilbereichen seiner Oberfläche eine antimikrobielle Beschichtung, bevorzugt eine Silberbeschichtung aufweist und/oder dass der Blasenkatheter (2) an seinem katheterspitzenfernen Ende ein das Ende verdickendes Blockierelement (8) aufweist.

## Claims

1. A catheter set (1) comprising a urinary catheter (2), with a flexible catheter shaft (5) and a catheter tip (6), and an elongate plastics film packaging (3), into which the urinary catheter (2) is inserted into a sealed catheter receiving chamber (10), whereby a reservoir (11) with an activating agent and/or a lubricant is formed by the packaging (3) adjacent the catheter tip end of the catheter receiving chamber (10), the reservoir (11) and the catheter receiving chamber (10) being formed by plastics weld seams (12, 13, 14) extending preferably substantially perpendicularly to the longitudinal direction of the plastics film packaging, and the reservoir (11) is formed by a first plastics weld seam (14), which separates the reservoir (11) from the catheter receiving chamber (10), and a second plastics weld seam (12), which seals the reservoir (11) at a free end of the packaging (3), **characterised in that** the plastics film packaging (3) is made from a plastics film tube, the second plastics weld seam (12) is stronger than the first plastics weld seam (14), such that in the event of pressure being exerted on the reservoir (11) only the first plastics weld seam (14) splits open to open the reservoir (11).

2. A catheter set according to claim 1, **characterised in that** a holding zone (20) is formed in the packaging (3) to the side in the region of the catheter tip end of the catheter receiving chamber (10), by which holding zone the catheter receiving chamber (10) is tapered in the direction of the reservoir (11) to form an outlet channel for the urinary catheter (2), the holding zone (20) being formed by a third plastics weld seam (21) in the plastics film tube forming the packaging (3).

3. A catheter set according to claim 2, **characterised in that** the holding zone (20) is formed in such a way by the third plastics weld seam (21) that the third plastics weld seam (21) forms a holding zone lumen which is airtight relative to the catheter receiving chamber (10), a predetermined breaking point preferably in the form of perforations (22) being formed in the material of the plastics film packaging forming the holding zone lumen to sever the material of the plastics film packaging forming the reservoir (11).

4. A catheter set according to claim 3, **characterised in that** a further predetermined breaking point preferably in the form of perforations (23) is formed in the material of the plastics film packaging forming the holding zone lumen to sever the material of the plastics film packaging forming the catheter receiving chamber (20) and adjoining the holding zone (20) in the direction of the end of the urinary catheter (2) remote from the catheter tip.

5. A catheter set according to any one of claims 1 to 4, **characterised in that** a severing zone (30) sealed in airtight manner relative to the catheter receiving chamber (10) is formed to the side in the packaging (3) in the region of the end of the catheter receiving chamber (10) remote from the catheter tip by a fourth plastics weld seam (31), a predetermined breaking point preferably in the form of perforations (32) being formed in the material of the plastics film packaging forming the severing zone (30) to sever the material of the plastics film packaging forming the end of the catheter receiving chamber (10) remote from the catheter tip.

6. A catheter set according to any one of claims 1 to 5, **characterised in that** the plastics film tube comprises an antimicrobial coating, preferably a silver coating, at least in sub-zones of its surface and/or **in that** the urinary catheter (2) comprises a blocking element (8) at its end remote from the catheter tip which makes the end thicker.

## Revendications

1. Ensemble cathéter (1), comprenant une sonde vésicale (2) avec une tige de cathéter souple (5) et avec une pointe de cathéter (6) ainsi qu'un emballage longitudinal en film plastique (3) dans lequel la sonde vésicale (2) est placée dans un espace de logement de cathéter fermé (10), un réservoir (11) avec un agent activant et/ou un agent lubrifiant étant conçu par l'emballage (3) adjacent à l'extrémité de l'espace de logement de cathéter (10) située du côté de la pointe de cathéter le réservoir (11) et l'espace de logement de cathéter (10) étant formés par des soudures de plastique (12, 13, 14) s'étendant de préférence globalement perpendiculairement au sens de la longueur de l'emballage en film plastique et le réservoir (11) étant formé par une première soudure de plastique (14) qui délimite le réservoir (11) par rapport à l'espace de logement de cathéter (10), et par une deuxième soudure de plastique (12) qui ferme le réservoir (11) à une extrémité libre de l'emballage (3), **caractérisé en ce que** l'emballage en film plastique (3) étant fabriqué en un tuyau de film plastique, la deuxième soudure de plastique (12) est conçue renforcée par rapport à la première soudure de plastique (14) de telle sorte que, lorsque l'on pousse sur le réservoir (11), seule la première soudure de plastique (14) se rompt pour ouvrir le réservoir (11).

2. Ensemble cathéter selon la revendication 1, **caractérisé en ce qu'**une zone de préhension (20) est conçue sur l'emballage (3), sur le côté, dans la zone de l'extrémité de l'espace de logement de cathéter (10) située du côté de la pointe de cathéter, zone de préhension par laquelle l'espace de logement de cathéter (10) est rétréci en direction du réservoir (11) pour former un canal de sortie pour la sonde vésicale (2), la zone de préhension (20) étant formée par une troisième soudure de plastique (21) sur le tuyau de film plastique constituant l'emballage (3).

3. Ensemble cathéter selon la revendication 2, **caractérisé en ce que** la zone de préhension (20) est formée par la troisième soudure de plastique (21) de telle sorte que la troisième soudure de plastique (21) forme un espace vide de zone de préhension étanche par rapport à l'air de l'espace de logement de cathéter (10), une zone de rupture conçue de préférence comme une perforation (22) étant prévue dans le matériau de l'emballage en film plastique qui forme l'espace vide de zone de préhension afin de séparer le matériau de l'emballage en film plastique qui forme le réservoir (11).

4. Ensemble cathéter selon la revendication 3, **caractérisé en ce qu'**une autre zone de rupture conçue de préférence comme une perforation (23) est réalisée dans le matériau de l'emballage en film plastique formant l'espace vide de zone de préhension afin de séparer le matériau de l'emballage en film plastique qui forme l'espace de logement de cathéter (20) et qui fait suite à la zone de préhension (20) en direction de l'extrémité, éloignée de la pointe de cathéter, de la sonde vésicale (2).

5. Ensemble cathéter selon l'une des revendications 1 à 4, **caractérisé en ce qu'**une zone de séparation (30) fermée de manière étanche par rapport à l'air de l'espace de logement de cathéter (10) est formée par une quatrième soudure de plastique (31) sur l'emballage (3), sur le côté, dans la zone de l'extrémité, éloignée de la pointe de cathéter, de l'espace de logement de cathéter (10), une zone de rupture conçue de préférence comme une perforation (32) étant formée dans le matériau de l'emballage en film plastique formant la zone de séparation (30) afin de séparer le matériau de l'emballage en film plastique qui forme l'extrémité, éloignée de la pointe de cathéter, de l'espace de logement de cathéter (10).

6. Ensemble cathéter selon l'une des revendications 1 à 5, **caractérisé en ce que** le tuyau de film plastique comporte au moins, dans certaines zones de sa surface, un revêtement antimicrobien, de préférence un revêtement en argent, et/ou **en ce que** la sonde vésicale (2) comporte à son extrémité éloignée de la pointe de cathéter un élément de blocage (8) épaississant l'extrémité.
